# EUROPEAN PATENT SPECIFICATION

(11) **EP 0 679 394 B1**
(45) Date of publication and mention of the grant of the patent: **14.01.1998**
(21) Application number: 95201046.0
(22) Date of filing: 24.04.1995
(51) Int. Cl.: A61K 31/12

(54) **Menatetronome derivative as antiarteriosclerotic agent**
Hematetrenome Derivat als antiarteriosklerotisches Mittel
Dérivé du Menatetrenome comme agent antiartériosclérotique

(30) Priority: 28.04.1994 JP 111684/94; 18.01.1995 JP 6049/95
(43) Date of publication of application: 02.11.1995
(73) Proprietor: Eisai Co., Ltd., Tokyo (JP)
(72) Inventor: Vermeer, Cees, NL-6200 MD Maastricht (NL)
(74) Representative: Van Someren, Petronella F. H. M.

(56) References cited:
- EP-A- 0 347 864
- ATHEROSCLEROSIS, vol. 116, no. 1, pages 117-123, K-S G. JIE ET AL. 'VITAMIN K INTAKE AND OSTEOCALCIN LEVELS IN WOMEN WITH AND WITHOUT AORTIC ATHEROSCLEROSIS: A POPULATION-BASED STUDY'
- CALCIFIED TISSUE INTERNATIONAL, vol. 54, no. 3, March 1994 pages 224-230, T.M. DOHERTY ET AL. 'CORONARY ARTERIAL CALCIFICATION AS AN ACTIVE PROCESS: A NEW PERSPECTIVE ON AN OLD PROBLEM'
- DIALOG FILE SUPPLIER : PASCAL NO. : 75-0001427, 'LA TENEUR DES VITAMINES E, K ET C DANS LE SERUM SANGUIN DES MALADES AVEC ATHEROSCLEROSE'

## Description

This invention relates to an agent for treating and ameliorating arteriosclerosis, for example, coronary arteriosclerosis, abdominal aortic sclerosis, arteriosclerosis obliterans, renal arteriosclerosis, carotid arteriosclerosis, ophthalmic arteriosclerosis and cerebral arteriosclerosis.

A circulatory disease proceeds under the reciprocal actions of thrombus formed on the arterial wall with sclerosing changes. In general, it proceeds slowly and the arterial wall is gradually thickened, thus causing topical arteriosclerosis. The arteriosclerosis thus formed is then proceeds to calcification. Thus the arterial calcification is the final symptom of arteriosclerosis and there has been found out no method for efficaciously treating the same.

Doherty et al. (Calcif. Tissue Int., 1994, 54 : 224-230) disclose a decrease of gamma-glutamate carboxylase (vitamin K dependant) activity in atherosclerotic arteries which could result in a decreased synthesis of Gla-containing proteins and a corresponding decrease in the capacity to transport calcium out of the vessel wall.

Since arteriosclerosis slowly proceeds as described above, neither any subjective symptom nor any objective finding is presented in the early stage thereof. As the disease proceeds, however, there appear symptoms including palpitation, breathlessness, cold feeling and pain in the limbs, a transient attack and dropsy followed by severe conditions such as a heart attack, cerebral hemorrhage, necrosis in the limbs, renal insufficiency, gait disorders and disturbance of sensation and motility. Thus it is a severe disease which might finally result in the death. Although cancer takes over the first place of the mortality statistics by cause of death in Japan, it can be said that arteriosclerosis ranks first as the cause of death, since most of cardiac diseases and cerebral diseases are caused thereby.

Thus arteriosclerosis is a disease which outbreaks at various sites in the body and is severe in the conditions or unfavorable in the prognosis. Thus it has been required to develop a clinically useful drug for treating or ameliorating arteriosclerosis.

There has been known no drug which is efficacious directly for arteriosclerosis hitherto and the only existing therapy therefor comprises administering a serum cholesterol-lowering agent (a lipid-lowering agent) or an antiplatelet aggregation agent (an antithrombotic agent) to thereby indirectly eliminate the risk factors of arteriosclerosis.

However the risk factors of arteriosclerosis are not limited to serum cholesterol and thrombus but involve various ones such as neutral fat, obesity, diabetes, hyperuricemia, immunologic diseases, ageing, hypertension, stress, the intake of stimulating substances, heredity, sex and racial characteristics. Therefore it is impossible in practice to eliminate all of these risk factors merely by administering a lipid-lowering agent or an antithrombotic agent.

Accordingly, there is no drug at present which has the efficacy of directly treating or ameliorating various types of arteriosclerosis and is excellent in safety. Thus it has been urgently required to develop a novel drug therefor.

The present inventors have conducted extensive studies on arteriosclerosis which sometimes results in the death for a long time and searched for a clinically useful drug for treating or ameliorating the same. As a result, they have unexpectedly found out that the above-mentioned object can be achieved by using a menatetrenone derivative represented by the following general formula as the antiarteriosclerotic agent, thus completing the present invention:

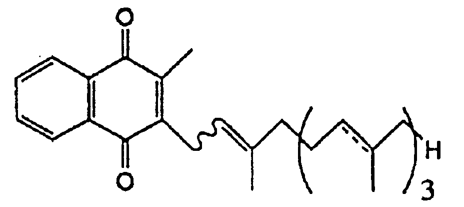

Accordingly, it is an object of the present invention to provide a novel drug being clinically useful in treating or ameliorating various types of arteriosclerosis such as coronary arteriosclerosis, abdominal aortic sclerosis, arteriosclerosis obliterans, renal arteriosclerosis, carotid arteriosclerosis, ophthalmic arteriosclerosis and cerebral arteriosclerosis, for which no clinically efficacious drug has been known hitherto, and excellent in safety. More particularly, the present invention relates to an antiarteriosclerotic agent which comprises a menatetrenone derivative as the active ingredient.

The invention provides use of a menatetrenone derivative represented by the following general formula (I) for manufacturing an antiarteriosclerotic agent: 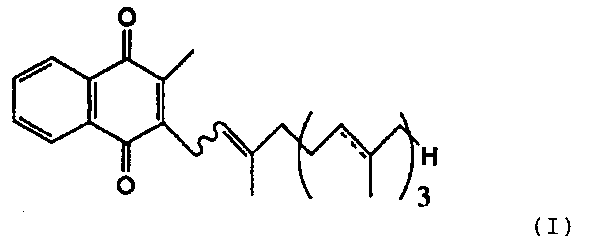 wherein the bond: -----
means a single or double bond.

It is preferable that the antiarteriosclerotic agent is therapeutically effective to at least one disease selected from among coronary arteriosclerosis, abdominal aortic sclerosis, arteriosclerosis obliterans, renal arteriosclerosis, carotid arteriosclerosis, ophthalmic arteriosclerosis and cerebral arteriosclerosis. The arteriosclerosis to cure by the invention may be at least one of the above shown diseases.

The invention provides a pharmacologically antiarteriosclerotic composition comprising a pharmacologically effective amount of the above defined menatetrenone compound having the formula (I) and a pharmacologically acceptable carrier.

The invention provides use of the menatetrenone compound as an antiarteriosclerotic agent.

The menatetrenone derivative to be used in the present invention is a compound represented by the following general formula:

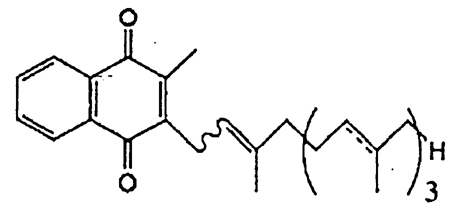

In the above formula, the bond represented by ----- means a single or double bond. 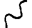 means an isomeric rotation, that is, the cis body or the trans body.

The menatetrenone derivative according to the present invention has a double bond or an asymmetric carbon atom in its molecule and sometimes occurs as geometrical isomers or optical isomers. In the present invention, either any of these isomers or a mixture of two or more of the same may be used without restriction. Particular examples of the menatetrenone derivative include the following ones, though it is needless to say that the present invention is not restricted thereto.
(1) 2-Methyl-3-[(2E,6E,10E)-3,7,11,15-tetramethyl-2,6,10,14-hexadecatetraenyl]naphthoquinone (generic name: vitamin K₂).
(2) 2-Methyl-3-[(2E)-3,7,11,15-tetramethyl-2-hexadecaenyl]naphthoquinone (generic name: vitamin K₁).
(3) 2-Methyl-3-[(2Z,6E,10E)-3,7,11,15-tetramethyl-2,6,10,14-hexadecatetraenyl]naphthoquinone (generic name: cis-vitamin K₂).
(4) 2-Methyl-3-[(2Z)-3,7,11,15-tetramethyl-2-hexadecaenyl]naphthoquinone (generic name: cis-vitamin K₁).

The compound of the present invention has been widely used in the clinical medicine as a compound having the effect of activating the mechanism of blood coagulation for treating newborn hypoprothrombinemia, intrapartum hemorrhage, hypoprothrombinemia occurring in the administration of an antibiotic, hypoprothrombinemia caused by biliary obliteration or bile secretion insufficiency, hypoprothrombinemia occurring in the administration of a coumarin anticoagulant, etc. Therefore it has been confirmed that this compound shows a high safety over prolonged administration. Also, an extremely low toxicity thereof has been confirmed by toxicity tests with the use of mice, rats and dogs.

Next, the results of the acute toxicity test of 2-methyl-3-[(2E,6E,10E)-3,7,11,15-tetramethyl-2,6,10,14-hexadecatetraenyl]naphthoquinone (generic name: vitamin K₂), which is a typical example of the compound of the present invention, will be given. An acute toxicity test was conducted as follows:

A single administration toxicity test was effected by orally, subcutaneously and intraperitoneally administering the test compound (medium: 5% gum arabic) to SD rats, ICR mice and beagles aged 7 to 8 weeks (5 females and 5 males in each case).

As results, the LD₅₀ (mg/kg) data are summarized in the following table.

**[Table 1]**

| Acute toxicity of vitamin K₂ (LD₅₀; mg/kg) | | | | | | |
|---|---|---|---|---|---|---|
| Administration route | Rat | | Mouse | | Beagle | |
| | M | F | M | F | M | F |
| oral | >5000 | >5000 | >5000 | >5000 | >3000 | >3000 |
| subcutaneous | >5000 | >5000 | >5000 | >5000 | >3000 | >3000 |
| intraperitoneal | >5000 | >5000 | >5000 | >5000 | >3000 | >3000 |

As Table 1 clearly shows, the compound of the present invention has high LD₅₀ values, i.e., an extremely high safety.

Examples of the administration form of the compound of the present invention include preparations for oral administration such as powders, fine powders, granules, tablets, coated tablets and capsules, those for topical use such as ointments and plasters, suppositories and injections. These preparations can be manufactured in the conventional manner with the use of pharmaceutical carriers commonly employed in the art.

More specifically, an oral preparation is manufactured by blending the menatetrenone derivative with fillers optionally together with other additives required such as antioxidants, binders, disintegrating agents, lubricants, coloring agents or corrigents and then shaping into powders, fine powders, granules, tablets, coated tablets, capsules, etc., in the conventional manner.

The fillers are exemplified by lactose, corn starch, sucrose, glucose, mannitol, sorbitol, crystalline cellulose and silicon dioxide. The binders are exemplified by polyvinyl alcohol, polyvinyl ether, methylcellulose, ethylcellulose, gum arabic, tragacanth, gelatin, shellac, hydroxypropylmethylcellulose, hydroxypropylcellulose, polyvinylpyrrolidone, polypropylene glycol/polyoxyethylene block polymer and meglumine. The disintegrating agents are exemplified by starch, agar, powdery gelatin, crystalline cellulose, calcium carbonate, sodium hydrogen carbonate, calcium citrate, dextrin, pectin and calcium carboxymethylcellulose. The lubricants are exemplified by magnesium stearate, talc, polyethylene glycol, silica and hardened vegetable oils. As the coloring agents, pharmaceutically acceptable ones may be used. The corrigents are exemplified by cocoa powder, menthol, aromatic powder, peppermint oil, Borneo camphor and cinnamon powder. As a matter of course, these tablets and granules may be optionally coated with sugar, etc., if necessary.

An injection is manufactured by blending the menatetrenone derivative with pH regulators, solubilizers, tonicity agents, etc., optionally together with other additives required such as dissolution aids, stabilizers and antioxidants, and then processing into the preparation in the conventional manner.

A preparation for topical use can be manufactured by any conventional method without restriction. Namely, the base material to be used for the preparation may be arbitrarily selected from among those commonly employed in drugs, quasi drugs, cosmetics, etc.

Particular examples of the base materials usable herein include animal and vegetable oils, mineral oils, ester oils, waxes, higher alcohols, fatty acids, silicone oils, surfactants, phospholipids, alcohols, polyhydric alcohols, water-soluble polymers, clay minerals and purified water, further may contain pH regulators, antioxidants, chelating agents, antiseptics, fungicides, coloring agents, perfumes, etc., if necessary, the base material of the preparation for topical use according to the present invention is not restricted thereto. Furthermore, it may contain additional ingredients such as blood flow accelerators, bactericides, antiinflammatory agents, cell potentiators, vitamins, amino acids, humectants and keratolytics, if desired. The base material is used in such an amount as to give a concentration commonly defined in the formulation of such a preparation for topical use.

The clinical dose of the menatetrenone derivative of the present invention is not restricted but varies depending on, for example, the conditions, the severity, the age and the complication. It also varies depending on the type of the salt and the administration route. In general, the dose of the compound ranges from 10 to 2,000 mg/day, preferably from 50 to 1,500 mg/day and still preferably from 100 to 1,000 mg/day, for an adult. It may be orally, intravenously, rectally or percutaneously administered.

To illustrate the usefulness of the compound of the present invention as an agent for treating and ameliorating arteriosclerosis, the following examples of pharmacological experiments will be given. However it is needless to say that neither the compound of the present invention nor the use of the same is restricted thereto.

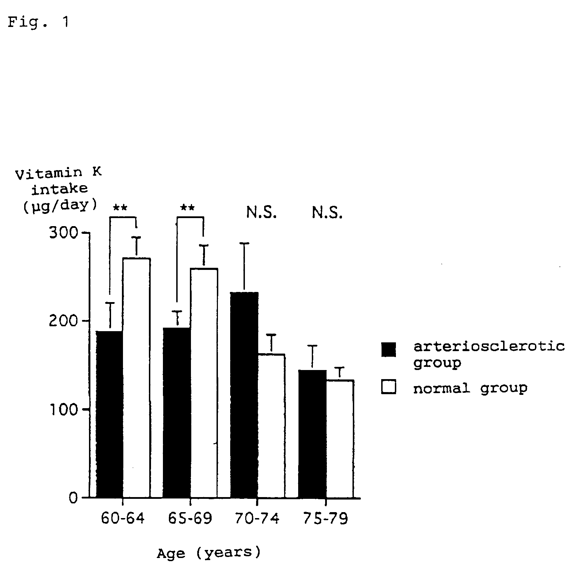

Fig. 1 is a graph which shows the relationship between the occurrence of abdominal aortic sclerosis and the vitamin K₂ intake in postmenopausal women classified by generation (expressed in "mean ± standard error").

Use is made of 2-methyl-3-[(2E,6E,10E)-3,7,11,15-tetramethyl-2,6,10,14-hexadecatetraenyl]naphthoquinone (generic name: vitamin K₂), which is a typical example of the compound of the present invention, as a test compound.

### Experiment 1: Evaluation of abdominal aortic sclerosis and vitamin K₂ intake in postmenopausal women

### (Method)

### (1) Evaluation of abdominal aortic sclerosis

Subjects were selected at random from among women aged 60 to 79. Calcification of the abdominal aorta of each subject was evaluated by two experts by the blind test method by taking the lateral X-ray photograph of the lumbara spine (T12-S1). The calcification indicated the existence of arteriosclerosis.

### (2) Evaluation of vitamin K₂ intake

Daily vitamin K₂ intake was determined by investigating the details of meals while focusing the attention to materials rich in vitamin K₂ and calculating the vitamin K₂ contents in the taken foods on the basis of various tables of food compositions.

### (Results)

Fig. 1 shows the relationship between the occurrence of arteriosclerosis and vitamin K₂ intake classified by generation (Fig. 1).

### Experiment 2: Effect of vitamin K₂ on arteriosclerotic rats

### (Method)

Male SD rats aged seven weeks were classified into seven groups as listed in the following table. Then vitamin D₂, vitamin D₂ with vitamin K₂ (in a low dose), and vitamin D₂ with vitamin K₂ (in a high dose) were given respectively to the groups A, B and C.

**[Table 2]**

| Group | n = | Vitamin D₂ (IU/kg/day) | Vitamin K₂ (mg/kg/day) | Administration period (weeks) |
|---|---|---|---|---|
| control | 8 | 0 | 0 | - |
| A | 8 | 2.5 × 10⁵; p.o. | 0 | 6 |
| B | 13 | ditto | 10; i.p. | 6 |
| C | 13 | ditto | 100; ditto | 6 |

After feeding for five weeks, the urine of each group was sampled for a period of 20 hours and the urinary calcium, phosphorus and creatinine levels were measured.

After feeding for six weeks, the animals of each group were bled to death and the blood, aorta (from the aortic arch to the thoracic aorta) and kidneys were sampled.

The serum was separated from the blood and the serum calcium and phosphorus levels were measured.

The aorta and kidneys were defatted, dehydrated and incinerated and the calcium and phosphorus contents therein were determined.

The calcium concentrations were determined by atomic absorption spectrophotometry. The inorganic phosphorus concentrations were determined by Goldenberg's method. The creatinine concentrations were determined by the enzymatic method.

### (Results)

The results are given in the following table. (The data of each group were compared with those of the control group according to the t-test method.)

| Sample | Item | Control | Group A | Group B | Group C |
|---|---|---|---|---|---|
| Aorta | Ca (mg/dl) | 0.4±0.07 | 11.6±11.1* | 5.0±3.8 | 2.6±3.2* |
| | P (mg/dl) | 0.7±0.1 | 10.9±7.1** | 12.6±12.2 | 3.7±4.3* |
| Kidney | Ca (mg/dl) | 0.3±0.03 | 7.6±7.8* | 3.4±3.8 | 1.6±1.4 |
| | P (mg/dl) | 3.1±0.3 | 7.2±2.0*** | 6.3±2.7 | 4.5±1.4* |
| Serum | Ca (mg/dl) | 9.5±0.7 | 9.8±1.4 | 9.9±1.4* | 11.0±1.4 |
| | P (mg/dl) | 7.4±0.7 | 8.2±1.4 | 8.1±0.5 | 8.0±0.6 |
| Urine | urine volume (ml) | 15.2±6.6 | 18.6±12.3 | 16.5±7.8 | 28.7±16.9 |
| | Ca (mg/dl) | 1.9±0.9 | 1.9±1.0 | 1.7±1.1 | 2.8±1.7 |
| | P (mg/dl) | 39.8±18.6 | 59.1±20.2 | 52.4±26.3 | 37.8±24.7* |
| | creatinine (mg/dl) | 72.9±42.4 | 77.2±22.8 | 75.5±32.8 | 49.8±28.9* |

| | | | | | |
|---|---|---|---|---|---|
| *: P<0.05, | | | | | |
| **: P<0.01, | | | | | |
| ***: P<0.001. | | | | | |

As Table 3 clearly shows, the group A to which vitamin D₂ had been given showed significant increases in the calcium and phosphorus contents in the aorta and suffered from the occurrence of arteriosclerosis. In the groups B and C to which vitamin K₂ had been given respectively in low and high doses, in contrast, increases in the calcium and phosphorus contents in the aorta were suppressed depending on the dose. In particular, the group C showed significant differences.

Similarly, the calcium and phosphorus contents in the kidneys were significantly increased and renal arteriosclerosis occurred. In the groups B and C to which vitamin K₂ had been given respectively in low and high doses, however, increases in the calcium and phosphorus contents in the kidneys were suppressed depending on the dose, similar to the case of the aorta.

On the other hand, no significant difference was observed among these groups in the serum calcium and phosphorus levels and urinary calcium, phosphorus and creatinine levels.

Based on these results, it is obvious that menatetrenone derivatives typically exemplified by vitamin K₂ closely relate to arteriosclerosis occurring at various sites and exert a suppressive effect thereon. Also these menatetrenone derivatives are excellent in safety. These facts indicate that the menatetrenone derivative according to the present invention can be expected as an antiarteriosclerotic agent which is highly useful in the clinical medicine.

## Claims

1. Use of a menatetrone derivative represented by the following general formula (I) 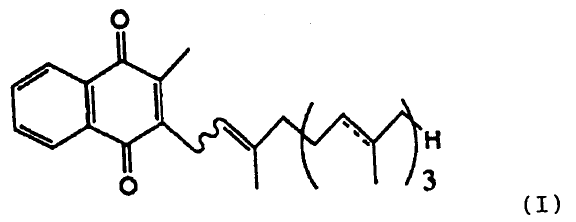 wherein the bond: -----
means a single or double bond
and wherein 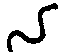 means an isomeric rotation,
for the manufacture of a medicament for the treatment of arteriosclerosis.

2. The use as claimed in Claim 1, in which the antiarteriosclerotic agent is therapeutically effective to at least one disease selected from among coronary arteriosclerosis, abdominal aortic sclerosis, arteriosclerosis obliterans, renal arteriosclerosis, carotid arteriosclerosis, ophthalmic arteriosclerosis and cerebral arteriosclerosis.

3. The use as claimed in Claim 1, in which the menatetrenone derivative is:
(1) 2-Methyl-3-[(2E,6E,10E)-3,7,11,15-tetramethyl-2,6,10,14-hexadecatetraenyl]naphthoquinone,
(2) 2-Methyl-3-[(2E)-3,7,11,15-tetramethyl-2-hexadecaenyl]naphthoquinone,
(3) 2-Methyl-3-[(2Z,6E,10E)-3,7,11,15-tetramethyl-2,6,10,14-hexadecatetraenyl]naphthoquinone or
(4) 2-Methyl-3-[(2Z)-3,7,11,15-tetramethyl-2-hexadecaenyl]naphthoquinone.

## Patentansprüche

1. Verwendung eines Menatetrenon-Derivats, dargestellt durch die folgende allgemeine Formel (I) 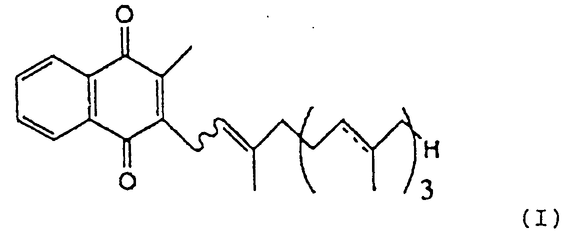 in der die Bindung -----
eine Einfach- oder Doppelbindung bedeutet
und in der 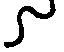 eine isomere Rotation bedeutet,
für die Herstellung eines Medikaments für die Behandlung von Arteriosklerose.

2. Verwendung nach Anspruch 1,
bei dem der antiarteriosklerotische Wirkstoff therapeutisch wirksam ist für mindestens eine Erkrankung, ausgewählt aus Koronar-Arteriosklerose, Abdominal-Aorta-Sklerose, Arteriosklerosis obliterans, Nierenarteriosklerose, Carotis-Arteriosklerose, Augenarteriosklerore und cerebrale Arteriosklerose.

3. Verwendung nach Anspruch 1,
bei der das Menatetrenon-Derivat
(1) 2-Methyl-3-[(2E,6E,10E)-3,7,11,15-Tetramethyl-2,6,10,14-Hexadecatetraenyl]naphthochinon,
(2) 2-Methyl-3-[(2E)-3,7,11,15-Tetramethyl-2-Hexadecaenyl]naphthochinon,
(3) 2-Methyl-3-[(2Z,6E,10E)-3,7,11,15-Tetramethyl-2,6,10,14-Hexadecatetraenyl]naphthochinon, oder
(4) 2-Methyl-3-[(2Z)-3,7,11,15-Tetramethyl-2-Hexadecaenyl]naphthochinon ist.

## Revendications

1. Utilisation d'un dérivé de ménatétrénone représenté par la formule générale (I) ci-après 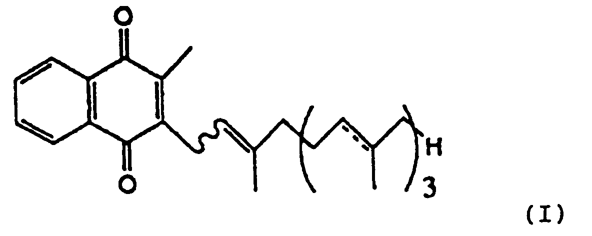 dans laquelle la liaison ----- représente une liaison simple ou double,
et 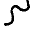 représente une rotation isomère, pour préparer un médicament destiné au traitement de l'artériosclérose.

2. Utilisation selon la revendication 1, dans laquelle l'agent anti-artériosclérotique présente une efficacité thérapeutique sur au moins une maladie choisie parmi l'artériosclérose coronarienne, la sclérose de l'aorte abdominale, l'artériosclérose oblitérante, l'artériosclérose rénale, l'artériosclérose carotidienne, l'artériosclérose ophtalmique, et l'artériosclérose cérébrale.

3. Utilisation selon la revendication 1, dans laquelle le dérivé de ménatétrénone est l'un des composés suivants :
(1) 2-méthyl-3-[(2E,6E,10E)-3,7,11,15-tétraméthyl-2,6,10,14-hexadécatétraényl]naphtoquinone,
(2) 2-méthyl-3-[(2E)-3,7,11,15-tétraméthyl-2-hexadécaényl]naphtoquinone,
(3) 2-méthyl-3-[(2Z,6E,10E)-3,7,11,15-tétraméthyl-2,6,10,14-hexadécatétraényl]naphtoquinone, ou
(4) 2-méthyl-3-[(2Z)-3,7,11,15-tétraméthyl-2-hexadécaényl]naphtoquinone.
